(19) Europäisches Patentamt
European Patent Office
Office européen des brevets

(11) Publication number: **0 040 896**
**B1**

(12) # EUROPEAN PATENT SPECIFICATION

(45) Date of publication of patent specification: **25.04.84**

(51) Int. Cl.[3]: **C 07 C 102/08**, C 07 C 103/22

(21) Application number: **81200767.2**

(22) Date of filing: **20.08.79**

(60) Publication number of the earlier application in accordance with Art. 76 EPC: **0008532**

(54) **Synthesis of amides.**

(30) Priority: **22.08.78 JP 102614/78**
**30.08.78 JP 106541/78**

(43) Date of publication of application:
**02.12.81 Bulletin 81/48**

(45) Publication of the grant of the patent:
**25.04.84 Bulletin 84/17**

(84) Designated Contracting States:
**BE CH DE FR GB IT NL SE**

(56) References cited:
**DE - B - 1 256 214**
**GB - A - 999 590**

**CHEMICAL ABSTRACTS, vol. 89, no. 3, 17th July 1978, page 623, no. 23994d Columbus, Ohio, U.S.A.**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Masuko, Fujio**
**2200, Oaza Tsurusaki**
**Ohita-shi (JP)**
Inventor: **Katsura, Fujio Sumitomo Chem. Co. Ltd.**
**Apt. 335 30, Korigaoka-8-chome**
**Hirakata-shi (JP)**

(74) Representative: **Harrison, David Christopher et al,**
**MEWBURN ELLIS & CO 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 040 896 B1

Synthesis of amides

This invention relates to a process for producing amides.

More particularly, the invention relates to an improved process for producing an amide of the formula (I),

$$R—CONH_2 \qquad (I)$$

wherein R is a straight or branched $C_1$—$C_6$ alkyl or alkenyl group, or a phenyl or aralkyl group, which said alkyl or alkenyl group is unsubstituted or substitued by a halogen atom, or an alkoxyl, alkylthio, dialkylamino, alkylsulfonyl, phenyl, phenoxy, phenylthio or hydroxy group, and which said phenyl or aralkyl group is unsubstituted or substituted by a halogen atom or an alkyl, alkenyl, alkoxyl, alkylthio, dialkylamino, alkylsulfonyl, phenyl, phenoxy, phenylthio or hydroxy group.

Amides within the formula (I) are important as intermediates for, e.g. medicines, agricultural chemicals, fine chemicals and dyestuffs. In particular, amides of the formula (I) can be converted to amines which are important as resolving agents in the production of optical isomers useful as medicines, agricultural chemicals or the like and also as intermediates in the production of medicines having excellent pharmacological effects such as the anti-cholesterol effect. A process for such conversion is described and claimed in European Patent Application No. 79301696.5 (Publication No. 0008532) from which this application is divided.

German AS 1256214 discloses a process for producing a specific amide, namely methacrylic acid amide by oxidative hydrolysis of methacrylic acid nitrile in the presence of an organic secondary amine (N,N'-di-sec-butyl p-phenylenediamine) as oxidation retardant.

Chemical Abstract 2399d (1978), *89*, 623 of Japanese unexamined patent publication No. 78.12805 discloses a process for the production of carboxamides by oxidative hydrolysis of cyano compounds in the presence of alkali metal, alkaline earth metal, or ammonium salts of transition metal oxides. This is a specific process which apparently allows oxidative hydrolysis of only one cyano group of a compound having two such groups, the other cyano group remaining intact (the specific example given being the conversion of $NCC(NH_2):C(CN)N:CHPh$ to $NCC(NH_2):C(CONH_2)N:CHPh$). The yield is, however apparently rather low and the reaction time extremely lengthy (71.2% and 16 hours respectively for the specific example given).

A previously known process for producing an amide of the formula (I) comprises heating the corresponding nitrile compound with an excess amount of hydrogen peroxide in an alcohol-water solvent in the presence of an alkali. However, this process requires the use of large amounts of hydrogen peroxide when carried out industrially and this carries with it the disadvantages of being uneconomical and dangerous.

We have made extensive investigations as to the conditions of hydrolysis of the nitrile to obtain the amide of the formula (I) and have found that the abovementioned disadvantages of known processes can be overcome by conducting the hydrolysis using a base and hydrogen peroxide in the presence of an organic quaternary ammonium salt and/or tertiary amine.

This process is advantageous from an industrial point of view in that the amount of hydrogen peroxide used can be decreased considerably and the reaction velocity becomes greater to permit the decrease of reaction time.

The present invention provides a process for preparing an amide compound of the formula (I)

$$R—CONH_2 \qquad (I)$$

wherein R is as defined above, which process comprises hydrolyzing a nitrile compound of the formula (II)

$$R—CN \qquad (II)$$

wherein R is as defined above, in the presence of a base and hydrogen peroxide using an organic quaternary ammonium salt and/or tertiary amine as a catalyst.

A nitrile compound of the formula (3),

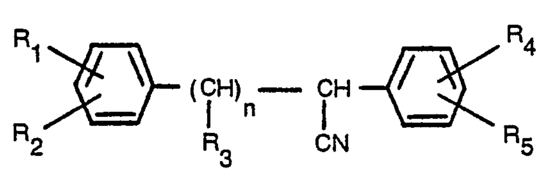

(3)

wherein $R_1$, $R_2$, $R_4$ and $R_5$ are each optionally present and are, independently of one another, a halogen atom, a hydroxy, trihalogenomethyl, phenyl, phenoxy or phenylthio group, or a straight or branched

$C_1$—$C_6$ alkyl, hydroxyalkyl, alkenyl, alkoxyl, alkylthio, dialkylamino or alkylsulfonyl group or either or both of ($R_1$ and $R_2$) and ($R_4$ and $R_5$) may, together with carbon atoms of a respective nucleus form a ring, $R_3$ is a hydrogen atom or any of the groups of $R_1$, $R_2$, $R_4$ and $R_5$, n is 1, 2 or 3 and, when n is 2 or 3, each $R_3$ may be the same as or different from the other or others, can be hydrolysed by the above process to obtain a compound of the formula (2)

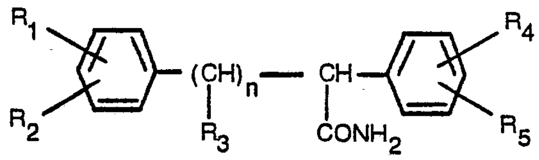

(2)

wherein $R_1$, $R_2$, $R_3$, $R_4$, $R_5$ and n are as defined above and this compound can be alkali-decomposed by the process described in the abovementioned European Patent Publication No. 0008532 to obtain a compound of the formula (1)

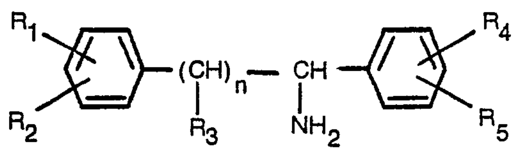

(1)

wherein $R_1$, $R_2$, $R_3$, $R_4$, $R_5$ and n are as defined above.

In carrying out the process of producing the amide of the formula (I), the compound of the formula (II) used as raw material in a process embodying the present invention, may, for example, be acetonitrile, propionitrile, butyronitrile, isobutyronitrile, isopropenylnitrile, benzonitrile, acrylonitrile, 2- or 3-methoxypropionitrile, 2- or 3-phenoxypropionitrile, 2- or 3-phenylthiopropionitrile, 2- or 3-methylthiopropionitrile, 2- or 3-dimethylaminopropionitrile, 2- or 3-hydroxypropionitrile, 2- or 3-methylsulfonylpropionitrile, 2- or 3-chloropropionitrile, 2- or 3-bromopropionitrile, 2- or 3-phenylpropionitrile, 2- or 3-p-tolylpropionitrile, 2,3-dimethoxypropionitrile, 2,3-diphenoxypropionitrile, 2,3-bis(phenylthio)-propionitrile, 2,3-bis(methylthio)-propionitrile, 2,3-bis(dimethylamino)-propionitrile, 2,3-bis(methylsulfonyl)-propionitrile, 2,3-dichloropropionitrile, 2,3-diphenylpropionitrile, 2-methoxy-3-ethoxypropionitrile, 2-phenoxy-3-phenylpropionitrile, 2-phenyl-3-(p-tolyl)-propionitrile, 2-phenyl-3-methylthiopropionitrile, 2-phenyl-3-hydroxypropionitrile, 2-chloro-3-bromopropionitrile, 2-methylsulfonyl-3-phenylpropionitrile, 3,3-diphenylpropionitrile, 3,3-diethoxypropionitrile, 3,3-bis(dimethylamino)-propionitrile, 2-phenyl-3,3-dichloropropionitrile, 2-(p-tolyl)-3,3-diphenylpropionitrile, 2,2-diphenyl-3-hydroxypropionitrile, 2,2,3-trichloropropionitrile, 2,3,3-triphenylpropionitrile, 2-phenyl-2-(p-tolyl)-3-chloropropionitrile, 2-phenylthioacetonitrile, 2,2-diphenylacetonitrile, 3-phenyl-4,4-dibromobutyronitrile, 2-phenyl-3-(p-tolyl)-acrylonitrile, 2,4-dichlorobenzonitrile and the like.

The hydrolysis is carried out in the presence or absence of a solvent. The solvents used for the reaction include, for example, water, alcohols such as methanol, ethanol, isopropanol, hexanol and butanols, t-butanols, benzene, toluene, xylene, chlorobenzenes, hexane, heptane, methyl cellosolve, dioxane, tetrahydrofuran, dimethylformamide, ether, dimethylsulfoxide and so on. These solvents are used alone, or in mixture. If the compound of the formula (II) is solid, it is preferred from operational viewpoint to select a solvent capable of dissolving the compound of the formula (II).

As the base to be used, alkali metals, alkali metal hydroxides, alkali metal carbonates, alkali metal alkoxides, alkaline earth metal hydroxides, alkali metal hydrides, etc. are illustrated. The amounts thereof to be used are arbitrarily selected from the range of 0.1 to 10 mols, preferably 0.1 to 2 mols per mol of the compound of the formula (II).

The amount of hydrogen peroxide to be used is arbitrarily in an amount over 1 mol, but it is sufficient in a range of about 2 to 3 mols per mol of the compound of the formula (II).

As the organic quaternary ammonium salt and tertiary amine to be used as catalyst, for example, tetra-n-butylammonium-chloride, -bromide or -hydroxide, tetraethylammonium-chloride, -bromide or -hydroxide, triethylbenzylammonium-chloride, -bromide or -hydroxide, triethylamine, trimethylamine, tributylamine, triisopropylamine, etc. are illustrated. These catalysts are used alone or in mixture. The amount of the catalyst to be used is arbitrarily selected in the range of 1/200 or 1/5 mol per mol of the raw compound of the formula (II), but is practically sufficient in an amount of about 1/100 mol per mol of the compound of the formula (II).

The reaction temperature is arbitrarily selected in the range of 0° to 300°C, preferably 20°C to 100°C.

With regard to the reaction pressure, there is no limitation in particular, and atmospheric pressure or increased pressure is used as well.

The reaction is carried out well both in liquid or vapor phase, and in batch or continuous system.

After completion of the reaction, the reaction mixture can be subjected to conventional aftertreatment such as separation, concentration, distillation, crystallization, etc., whereby the desired compound of the formula (I) is obtained in good yield.

As the compound of the formula (I) thus obtained are illustrated in the following; for example, acetamide, propionamide, butyramide, isobutyramide, isopropenylamide, benzamide, acrylamide, 2- or 3-methoxypropionamide, 2- or 3-phenoxypropionamide, 2- or 3-phenylthiopropionamide, 2- or 3-methylthiopropionamide, 2- or 3-dimethylaminopropionamide, 2- or 3-hydroxypropionamide, 2- or 3-methylsulfonylpropionamide, 2- or 3-chloropropionamide, 2- or 3-bromopropionamide, 2- or 3-phenylpropionamide, 2- or 3-p-tolylpropionamide, 2,3-dimethoxypropionamide, 2,3-diphenoxypropionamide, 2,3-bis(phenylthio)-propionamide, 2,3-bis(methylthio)-propionamide, 2,3-bis(dimethylamino)-propionamide, 2,3-bis(methylsulfonyl)-propionamide, 2,3-dichloropropionamide, 2,3-diphenylpropionamide, 2-methoxy-3-ethoxypropionamide, 2-phenyl-3-phenylpropionamide, 2-phenyl-3-(p-tolyl)-propionamide, 2-phenyl-3-methylthiopropionamide, 2-phenyl-3-hydroxypropionamide, 2-chloro-3-bromopropionamide, 2-methylsulfonyl-3-phenylpropionamide, 3,3-diphenylpropionamide, 3,3-diethoxypropionamide, 3,3-bis(dimethylamino)-propionamide, 2-phenyl-3,3-dichloropropionamide, 2-(p-tolyl)-3,3-diphenylpropionamide, 2,2-diphenyl-3-hydroxy-propionamide, 2,2,3-trichloropropionamide, 2,3,3-triphenylpropionamide, 2-phenyl-2-(p-tolyl)-3-chloropropionamide, 2-phenylthioacetamide, 2,2-diphenylacetamide, 3-phenyl-4,4-dibromobutyramide, 2-phenyl-3-(p-tolyl)-acrylamide, 2,4-dichlorobenzamide and the like.

The invention is illustrated by way of example in the following with an intention only for illustration and without any intention to add any limitations to the invention. In Examples, percents are by weight.

Production of amide derivatives of the formula (I)

Examples 1 to 3

221.3 Grams of 2-phenyl-3-(p-tolyl)-propionitrile (II—1), 40 g of a 25% aqueous sodium hydroxide solution, 291.5 g of a 35% aqueous hydrogen peroxide solution and 3.22 g of tetra-n-butylammoniumbromide were mixed at 50°C for 4 hours in 663.9 g of methanol. After the reaction was completed, methanol was distilled off. The formed precipitates were filtered out, and washed with water to obtain 234.5 g of 2-phenyl-3-(p-tolyl)-propionamide (m.p.: 150°—151°C) (I—1) in a yield of 98%.

Using 2-phenylpropionitrile and acrylonitrile as raw materials, the reactions were carried out as in the above to obtain the amide compounds. The results were obtained as shown in the following Table 1.

Further, in comparative examples, reactions using no catalyst (organic quaternary ammonium salt) were carried out and the amides were prepared. The results are also shown in Table 1.

TABLE 1

| | Raw Material | Catalyst | Reaction Hour | Yield |
|---|---|---|---|---|
| Example 1 | 2-phenyl-3-(p-tolyl)-propionitrile | tetra-n-butylammonium bromide | 4 | 98% |
| Example 2 | 2-phenyl-propionitrile | tetra-n-butylammonium chloride | 4 | 97% |
| Example 3 | acrylonitrile | triethyl-benzyl-ammonium chloride | 4 | 98% |
| Comparative Example 1 | 2-phenyl-3-(p-tolyl)-propionitrile | none | 15 | 90% |
| Comparative Example 2 | 2-phenyl-propionitrile | none | 10 | 89% |
| Comparative Example 3 | acrylonitrile | none | 7 | 91% |

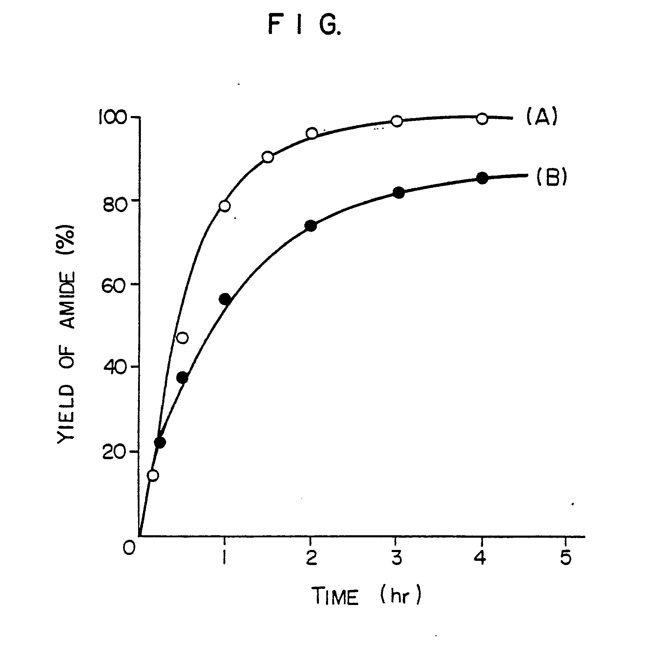

In comparison of Example 1 with Comparative Example 1, the relations between the reaction time and yield are shown in the attached Figure. In the Figure, (A) shows the result of Example 1 and (B) that of Comparative Example 1.

Example 4

221.3 Grams of 2-phenyl-3-(p-tolyl)-propionitrile (II—1), 40 g of a 25% aqueous sodium hydroxide solution, 291.5 g of a 35% aqueous hydrogen peroxide solution and 2.78 g of tetra-n-butylammonium chloride were mixed at 50°C for 4 hours in 663.9 g of methanol. After the reaction was completed, methanol was distilled off and the formed precipitate was filtered out and washed with water to obtain 234.5 g of 2-phenyl-3-(p-tolyl)-propionamide (m.p.: 150°—151°C) (I—1) in a yield of 98%.

Example 5

117.2 Grams of phenylacetonitrile (II—2), 56.1 g of a 25% aqueous potassium hydroxide solution, 291.5 g of a 35% aqueous hydrogen peroxide solution and 1.78 g of triethylbenzylammonium chloride were mixed at 50°C for 4 hours in 351.6 g of isopropanol. After completion of the reaction, isopropanol was distilled off and the formed precipitate was filtered out and washed with water to obtain 128.5 g of phenylacetamide (m.p.: 155°C) (I—2) in a yield of 95%.

Example 6

41.1 Grams of acetonitrile (II—3), 40 g of a 25% aqueous sodium hydroxide solution, 291.5 g of a 35% aqueous hydrogen peroxide solution and 1.01 g of triethylamine were mixed at 50°C for 4 hours. After the reaction was completed, the formed precipitate was filtered out, and washed with water to obtain 56.1 g of acetamide (m.p.: 81°C) (I—3) in a yield of 95%.

Example 7

103.1 Grams of benzonitrile (II—4), 9.51 g of sodium methoxide, 291.5 g of a 35% aqueous hydrogen peroxide solution and 2.78 g of tetra-n-butylammonium chloride were mixed in 309.3 g of methanol. After the completion of the reaction, the formed precipitate was filtered out and washed with water to obtain 116.3 g of benzamide (m.p.: 130°C) (I—4) in a yield of 96%.

Examples 8 to 23

Using several kinds of nitriles, the corresponding amides were prepared in the same manner as in Examples 4 to 7. The results are shown in Table 2

TABLE 2

| Example No. | Raw Material | Reaction Condition | Yield |
|---|---|---|---|
| 8 | 2-phenyl-3-methylpropionitrile | Same as Example 4 | 98% |
| 9 | 3,3-diphenylpropionitrile | Same as Example 4 | 99% |
| 10 | 2-(p-tolyl)-3,3-diphenylpropionitrile | Same as Example 4 | 98% |
| 11 | 2,2-diphenylacetonitrile | Same as Example 4 | 97% |
| 12 | 2,4-dichlorobenzonitrile | Same as Example 5 | 95% |
| 13 | 2-phenyl-3-(p-tolyl)acrylonitrile | Same as Example 5 | 96% |
| 14 | 2,3-dichloropropionitrile | Same as Example 5 | 95% |
| 15 | 2,3-diphenylpropionitrile | Same as Example 5 | 94% |
| 16 | 2-chloropropionitrile | Same as Example 6 | 94% |
| 17 | 3-hydroxypropionitrile | Same as Example 6 | 95% |
| 18 | butyronitrile | Same as Example 6 | 96% |
| 19 | 2-hydroxypropionitrile | Same as Example 6 | 95% |
| 20 | 3-phenyl-4,4-dibromobutyronitrile | Same as Example 7 | 96% |
| 21 | 2-phenoxy-3-phenylpropionitrile | Same as Example 7 | 97% |
| 22 | 2,3-bis(phenylthio)-propionitrile | Same as Example 7 | 96% |
| 23 | 3-(p-tolyl)-propionitrile | Same as Example 7 | 95% |

**Claims**

1. A process for preparing an amide represented by the general formula (I),

$$R—CONH_2 \qquad (I)$$

wherein R is a straight or branched $C_1$—$C_6$ alkyl or alkenyl group, or a phenyl or aralkyl group, which said alkyl or alkenyl group is unsubstituted or substitued by a halogen atom, or an alkoxyl, alkylthio,

dialkylamino, alkylsulfonyl, phenyl, phenoxy, phenylthio or hydroxy group, and which said phenyl or aralkyl group is unsubstituted or substituted by a halogen atom or an alkyl, alkenyl, alkoxyl, alkylthio, dialkylamino, alkylsulfonyl, phenyl, phenoxy, phenylthio, or hydroxy group by hydrolysis, in the presence of a base and hydrogen peroxide, of a nitrile of the formula (II),

$$R—CN \qquad (II)$$

wherein R is as defined above, characterized in that the reaction is carried out in the presence of an organic quaternary ammonium salt and/or a tertiary amine as a catalyst.

2. A process according to claim 1, wherein the said nitrile of formula (II) is a nitrile compound of the formula (3),

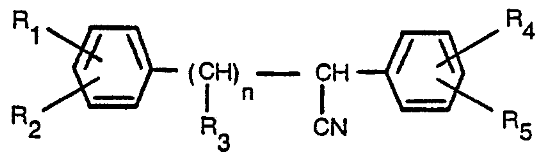

(3)

wherein $R_1$, $R_2$, $R_4$ and $R_5$ are each optionally present and are, independently of one another, a halogen atom, a hydroxy, trihalogenomethyl, phenyl, phenoxy or phenylthio group, or a straight or branched $C_1$—$C_6$ alkyl, hydroxyalkyl, alkenyl, alkoxyl, alkylthio, dialkylamino or alkylsulfonyl group, or either or both of ($R_1$ and $R_2$) and ($R_4$ and $R_5$) may, together with carbon atoms of a respective nucleus, form a ring, $R_3$ is a hydrogen atom or any of the groups of $R_1$, $R_2$, $R_4$ or $R_5$ and n is 1, 2 or 3 and, when n is 2 or 3, each $R_3$ may be the same as or different from the other or others, and the said amide of the formula (I) thus produced is an amide of the formula (2)

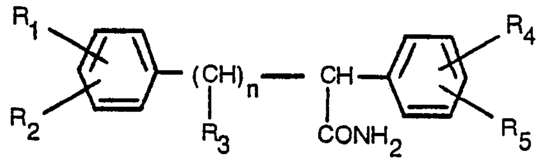

(2)

wherein $R_1$, $R_2$, $R_3$, $R_4$, $R_5$ and n are as defined above.

**Revendications**

1. Procédé de préparation d'un amide répondant à la formule générale (I):

$$R—CONH_2 \qquad (I)$$

dans laquelle R représente un groupe alcoyle ou alcényle en $C_1$—$C_6$, à chaîne droite ou ramifiée, ou un groupe phényle ou aralcoyle, ledit groupe alcoyle ou alcényle étant non substitué ou substitué par un atome d'halogène ou un groupe alcoxy, alcoylthio, dialcoylamino, alcoylsulfonyle, phényle, phénoxy, phénylthio ou hydroxy et ledit groupe phényle ou aralcoyle étant non substitué ou substitué par un atome d'halogène ou un groupe alcoyle, alcényle, alcoxy, alcoylthio, dialcoylamino, alcoylsulfonyle, phényle, phénoxy, phénylthio ou hydroxy, par hydrolyse, en présence d'une base et de peroxide d'hydrogène, d'un nitrile de formule (II):

$$R—CN \qquad (II)$$

dans laquelle R est tel que défini ci-dessus, caractérisé en ce que la réaction est effectuée en présence d'un sel d'ammonium quaternaire organique et/ou d'une amine tertiaire, agissant comme catalyseur.

2. Procédé selon la revendication 1, dans lequel ledit nitrile de formule (II) est un composé de formule (3):

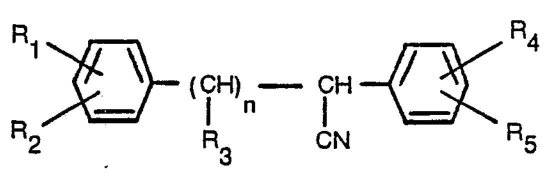

(3)

dans laquelle $R_1$, $R_2$, $R_4$ et $R_5$ sont chacun de présence facultative et représentent, indépendamment l'un de l'autre, un atome d'halogène, un groupe hydroxy, trihalogénométhyle, phényle, phénoxy ou phénylthio, ou un groupe à chaîne droite ou ramifiée en $C_1$—$C_6$ alcoyle, hydroxyalcoyle, alcényle, alcoxy, alcoylthio, dialcoylamino ou alcoylsulfonyle, ou bien l'un de ou l'un et l'autre de ($R_1$ et $R_2$) et ($R_4$ et $R_5$) peuvent former un cycle, ensemble avec l'atome de carbone du noyau correspondant, $R_3$ représente un atome d'hydrogène ou l'un quelconque des groupes $R_1$, $R_2$, $R_4$ et $R_5$ et n représente la valeur 1, 2 ou 3 et, lorsque n représente la valeur 2 ou 3, chaque $R_3$ peut être identique à ou différent de l'autre ou des autres, et ledit amide de formule (I) ainsi obtenu est un amide de formule (2):

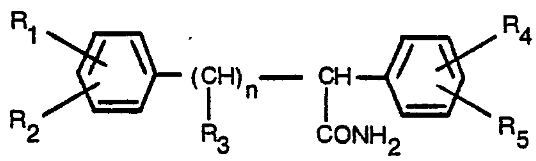

(2)

dans laquelle $R_1$, $R_2$, $R_3$, $R_4$, $R_5$ et n sont tels que défini ci-dessus.

## Patentansprüche

1. Verfahren zur Herstellung eines Amides der allgemeinen Formel (I):

$$R—CONH_2 \qquad (I)$$

worin R für eine unverzweigte oder verzweigte Alkyl- oder Alkenylgruppe mit 1 bis 6 Kohlenstoffatomen oder eine Phenyl- oder Aralkylgruppe steht, wobei die Alkyl- oder Alkenylgruppe unsubstituiert oder durch ein Halogenatom oder eine Alkoxy-, Alkylthio-, Dialkylamino-, Alkylsulfonyl-, Phenyl-, Phenoxy-, Phenylthio- oder Hydroxylgruppe substituiert ist und wobei die Phenyl- oder Aralkylgruppe unsubstituiert oder durch ein Halogenatom oder eine Alkyl-, Alkenyl-, Alkoxy-, Alkylthio-, Dialkylamino-, Alkylsulfonyl-, Phenyl-, Phenoxy-, Phenylthio- oder Hydroxylgruppe substituiert ist, durch Hydrolyse eines Nitrils der Formel (II):

$$R—CN \qquad (II)$$

worin R die obige Bedeutung hat, in Gegenwart einer Base und von Wasserstoffperoxid, dadurch gekennzeichnet, dass man die Reaktion in Gegenwart eines organischen quaternären Ammoniumsalzes und/oder eines tertiären Amins als Katalysator ausführt.

2. Verfahren nach Anspruch 1, dadurch gekennzeichnet, dass das Nitril der Formel (II) eine Nitrilverbindung der Formel (3):

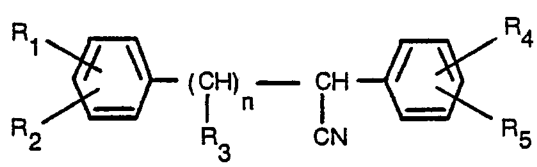

(3)

ist, worin $R_1$, $R_2$, $R_4$ und $R_5$ jeweils gegebenenfalls vorhanden sind und unabhängig voneinander ein Halogenatom, eine Hydroxyl-, Trihalogenmethyl-, Phenyl-, Phenoxy- oder Phenylthiogruppe oder eine unverzweigte oder verzweigte, 1 bis 6 Kohlenstoffatome enthaltende Alkyl-, Hydroxyalkyl-, Alkenyl-, Alkoxy-, Alkylthio-, Dialkylamino- oder Alkylsulfonylgruppe sind oder ($R_1$ und $R_2$) und/oder ($R_4$ und $R_5$) zusammen mit Kohlenstoffatomen eines entsprechenden Kernes einen Ring bilden können, $R_3$ ein Wasserstoffatom oder eine beliebige der Gruppen $R_1$, $R_2$, $R_4$ oder $R_5$ ist und n für 1, 2 oder 3 steht und, wenn n für 2 oder 3 steht, die einzelnen Substituenten $R_3$ gleich wie der andere oder die anderen oder verschieden von dem anderen oder den anderen sein können, und dass das so erzeugte Amid der Formel (I) ein Amid der Formel (2):

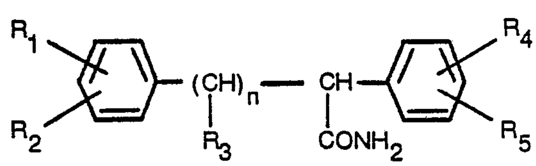

(2)

ist, worin $R_1$, $R_2$, $R_3$, $R_4$, $R_5$ und n die obigen Bedeutungen haben.

FIG.